# EUROPEAN PATENT APPLICATION

(11) **EP 1 719 469 A1**
(43) Date of publication of application: **08.11.2006**
(21) Application number: 06113372.4
(22) Date of filing: 02.05.2006
(51) Int. Cl.: A61B 17/80, A61B 17/00, A61B 17/86, A61B 17/16

(54) **Bone plate with intra-operatively tappable holes**

(30) Priority: 06.05.2005 FI 20055214
(71) Applicant: Inion Oy, 33520 Tampere (FI)
(72) Inventor: Pitkänen, Heikki, 33540, Tampere (FI); Happonen, Harri, 33820, Tampere (FI); Vuorisalo, Vesa, 33520, Tampere (FI)
(74) Representative: Kaukonen, Juha Veikko

(57) **Abstract**

A plate, its use, a system and an attachment method of the plate. The plate (1) comprises a first part (9) intended for attaching to at least one first bone fragment, and a second part (10) intended for attaching to at least one second bone fragment, and an upper surface (2) and a lower surface (3). The upper surface (2) comprises a plurality of holes, of which holes some are preholes (4), in at least one of which preholes an actual, threaded attachment hole (13) is arranged to be prepared *in situ,* and at least one free hole (5,7), which is a through hole extending from the upper surface (2) to the lower surface (3), the smallest diameter of the free hole (5,7) being larger than the diameter of the preholes (4).

## Description

### BACKGROUND OF THE INVENTION

The invention relates to a plate for fixing bone fragments to one another, the plate comprising a first part intended for attaching to at least one or more first bone fragments and a second part intended for attaching to one or more second bone fragments, an upper surface and a lower surface.

The invention also relates to the use of the plate.

The invention further relates to a system for fixing bone fragments to one another.

The invention still further relates to a method for fixing bone fragments to one another.

The wrist and the bones meeting therein may fracture, for instance, as a result of falling or stumbling. Very often it is the radius bone meeting the wrist joint that fractures such that its distal part breaks in one or more fragments at least partly off the shaft of the radius bone. The most typical one of these fractures is the so-called Colles' fracture. Commonly known plates, particularly intended for the treatment of the distal part of the radius, are used for treating these fractures. When such a plate is used the loose distal fragments are fitted into place in the shaft of the radius and the plate is attached over the fracture area such that its distal part is attached to the distal bone fragments off the radial shaft and correspondingly the proximal part is attached to the shaft of the radius.

Typically the plate is secured with screws to the shaft of the radius and with screws or pins to its distal part. Said screws or pins are fitted in holes drilled in the distal part, which holes are optionally arranged in different directions from one another. Plates of this kind have been disclosed, for instance, in US 2003/0105461 and US 6,358,250. In general, the plate is made of metal or plastic.

Plates as described above may also be used for treating osteotomies, i.e. in operations, in which a bone, for instance the radius, is intentionally cut to change its shape or position, for instance. In addition, the plates may be used for bone fusions, i.e. bone implantations.

The known plates involve some problems. First, it is difficult to attach the plate in correct position to bone fragments to be fixed to one another, because it is difficult to hold the plate steady on the bone while the attachment holes are being drilled. Another problem relates to the attachment of the plate to the bone. Namely, the tension of the attachment screws pressing the plate against the bone reduces by the effect of tension relaxation, in particular if the attachment screw is made of plastic. As the tension reduces the support of the plate to the bone fragments reduces, which may permit movement between the bone fragments. This delays healing of the fracture.

### BRIEF DESCRIPTION OF THE INVENTION

The object of the invention is to provide a novel and improved plate, its use, a system comprising the plate and a plate attachment method, by which the above problems will be reduced.

The plate of the invention is characterized in that the upper surface comprises a plurality of holes, of which some are preholes, in at least one of which preholes an actual, threaded attachment hole is arranged to be prepared *in situ,* and at least one free hole, which is a through hole extending from the upper surface to the lower surface, the smallest diameter of the free hole being larger than the diameter of the preholes.

The use of the plate in accordance with the invention is characterized by being used for fixing a distal radial fracture or osteotomy.

The system in accordance with the invention is characterized by including a plate, which comprises a first part intended for attaching to at least one first bone fragment, and a second part intended for attaching to at least one second bone fragment, an upper surface and a lower surface, the upper surface comprising a plurality of holes, of which holes some are preholes, and at least one free hole, which is a through hole extending from the upper surface to the lower surface, the smallest diameter of the free hole being larger than the diameter of the preholes, the system further including an attachment screw comprising a threaded portion, whose outer diameter is at most equal in size with the smallest diameter of the free hole but larger than the diameter of the prehole.

The method in accordance with the invention is characterized by using a plate, which comprises a first part to be attached to at least one first bone fragment, and a second part to be attached to at least one second bone fragment, an upper surface and a lower surface, the upper surface comprising a plurality of holes, of which holes some are preholes, and at least one free hole, which is a through hole extending from the upper surface to the lower surface, the smallest diameter of the free hole being larger than the diameter of the preholes, and attachment screws comprising a threaded portion, whose outer diameter is at most equal in size with the smallest diameter of the free hole and by arranging the plate on the bone, providing the bone, through the free hole, with a preattachment hole comprising a threaded portion provided in the bone, fitting the attachment screw through the free hole to the pre-attachment hole, providing one or more preholes with an actual attachment hole, which comprises the threaded plate portion and the threaded bone portion which is parallel and coaxial therewith and which extends inside the bone, and driving the attachment screw in the attachment hole.

The basic idea of the invention is that the plate includes preholes for attachment holes, through which preholes actual threaded attachment holes are prepared *in situ,* in which actual attachment holes there are fitted attachment screws for attaching the plate to bone fragments to be fixed to one another, and that the plate also includes at least one free hole, through which said attachment screw may pass without its thread locking in the plate.

The invention has an advantage that thanks to the free hole it is easy and quick to provide the plate with an attachment screw that presses the plate tightly against the bone and that through the preholes it is possible to prepare an attachment hole having a continuous thread extending from the plate to the inside of the bone. The continuous thread locks the screw not only in the bone but also in the plate, whereby the attachment of the plate is particularly firm and tight.

The basic idea of an embodiment of the plate in accordance with the invention is that a prehole is a through hole extending from the upper surface of the plate to the lower surface thereof. There is an advantage that it is very easy to provide the through hole with an attachment hole whose central axis is co-centric with the central axis of the prehole.

The basic idea of a second embodiment of the plate in accordance with the invention is that the plate comprises at least two attachment holes aligned in the transverse direction of the plate in the end of the second part that is closest to the first part. There is an advantage that the plate's attachment will be as firm as possible close to the fracture line.

The basic idea of a third embodiment of the plate in accordance with the invention is that a free hole is an elongated free hole whose diameter in one direction is larger than in another direction and whose smallest diameter is larger than the diameter of the preholes.

The basic idea of an embodiment of the system in accordance with the invention is that the thread in the threaded portion of an attachment screw is arranged to vary such that the thread crest is higher in the portion closer to the screw tip than in the portion further away from the tip. There is an advantage that a high crest attaches optimally to the spongy bone whereas a lower crest attaches optimally to the cortical bone.

The basic idea of an embodiment of the method in accordance with the invention is that an actual attachment hole of the plate is prepared at an angle to the plate, which angle differs from that of the prehole of the attachment hole to the plate. There is an advantage that the attachment screw can be arranged in such a position with respect to the plate that the prehole does not definitely determine the direction of the attachment screw, but the screw and consequently the plate can be attached to the bone tissue in the best possible manner in view of the medical and operational criteria associated with the procedure.

The basic idea of a second embodiment of the method in accordance with the invention is that originally the plate is only provided with preholes and that a free hole is not prepared in a prehole until the most advantageous location for the free hole has been found from the viewpoint of bone fragments to be fixed to one another.

### BRIEF DESCRIPTION OF THE DRAWINGS

Some embodiments of the invention are described in greater detail in connection with the attached drawings, in which
Figure 1 is a schematic view of a plate in accordance with the invention seen in the direction of the upper surface;
Figure 2 is a schematic side view of the plate of Figure 1;
Figure 3 is a schematic perspective view of the plate of Figure 1 seen in the direction of the upper surface;
Figure 4 is a schematic view of a detail in the system in accordance with the invention in partial cross section;
Figure 5 is a schematic view of a second plate in accordance with the invention seen in the direction of the upper surface; and
Figure 6 is a side view of the plate of Figure 5.

For the sake of clarity, the invention is shown in a simplified manner in the figures. Like reference numerals refer to like parts in the figures.

### DETAILED DESCRIPTION OF SOME EMBODIMENTS OF THE INVENTION

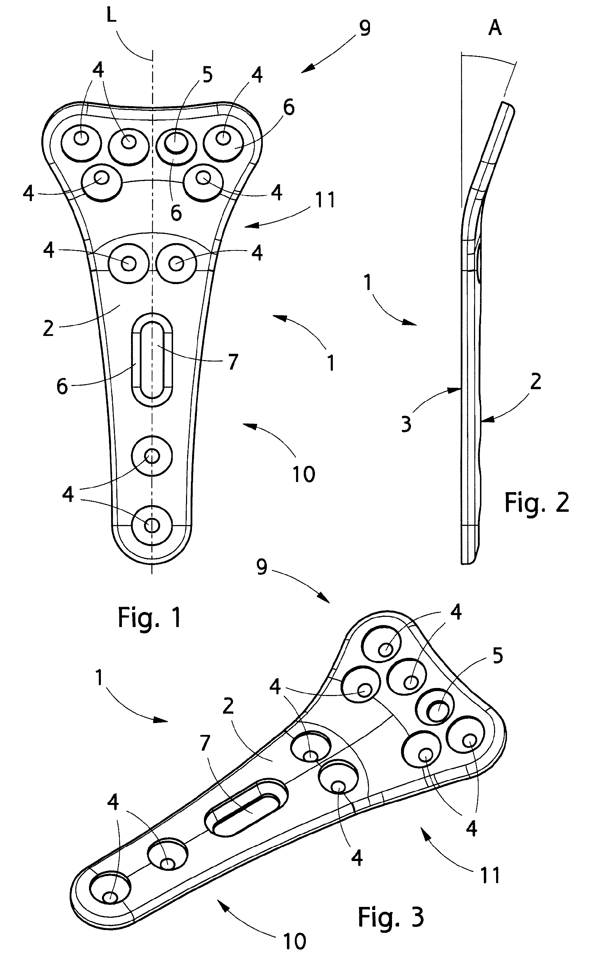

Figure 1 shows schematically a plate in accordance with the invention seen in the direction of the upper surface, Figure 2 is a schematic side view of the same plate and Figure 3 is a perspective view in the direction of the upper surface.

The plate 1 is intended for fixing a distal part loose from the shaft of the radius bone to the shaft of the radius bone such that the plate 1 is attached to the palm side of the radius bone. Depending on the type of the fracture there may be one or more distal bone fragments to be attached to the shaft of the radius bone. In connection with the following figures an operation like this will be described, but it is obvious that the plate, the arrangement and the method in accordance with the invention may also be used in treating other bone fractures, osteotomies and fusions.

The plate 1 is most preferably manufactured of biodegradable polymer material absorbable in the body that is made by polymerizing or copolymerizing, for instance, lactic acid, L-lactide, D-lactide, D,L-lactide, mesolactide, glycolic acid, glycolide or any cyclic ester copolymerizable with a lactide, or of any corresponding material known per se to the person skilled in the art, which materials will not be described herein in any greater detail. Other suitable biodegradable polymers, copolymers and polymer blends appear, for instance, in the following publications:
"Encyclopedic Handbook of Biomaterials and Bioengineering, Part A", Donald L. Wise, Debra J. Tarantolo, David E. Altobelli, Michael J. Yaszem-ski, Joseph D. Gresser, Edith R. Schwartz, 1992, Marcel Dekker, Inc., p. 977-1007;
"Biodegradable fracture-fixation devices in maxillofacial surgery", R. Suuronen, Int. J. Oral Maxillofac. Surgery, 1993, 22:50-57;
"Critical Concepts of Absorbable Internal Fixation", William S. Pietrzak, Portland Bone Symposium, Portland, Oregon, August 4-7, 1999;
"High-impact poly(L/D-lactide) for fracture fixation: in vitro degradation and animal pilot study", Jan Tams, Cornelis A.P. Joziasse, Ruud R.M. Bos, Fred R. Rozema, Dirk W. Grijpma and Albert J. Pennings, Biomaterials 1995, vol. 16, no. 18, p. 1409-1415;
"A Review of Material Properties of Biodegradable and Bioresorbable Polymers and Devices for GTR and GBR Applications", Dietmar Hutmacher, Markus B. Hürzeler, Henning Schliephake, The International Journal of Oral & Maxillofacial Implants, vol. 11, no. 5, 1996, p. 667-678; and
"Orthopaedic Application for PLA-Pga Biodegradable Polymers", Kyriacos A. Athanasiou, Mauli Agrawal, Alan Barber, Stephen S. Burkhart, The Journal of Arthroscopic and Related Surgery, vol. 14, no. 7 (October) 1988; 726-737.

It is further obvious to the person skilled in the art that the material may be a so-called composite material which consists of two or more materials or monomers, polymer chains, whose substantial feature is that they dissolve in the body. The composite material may contain bioglass, bioceramics, biologically active components, drugs, such as antibiotic or growth factor, and so on.

The manufacturing material of the plate may also contain softening agents, such as pyrrolidone softeners. Useful pyrrolidones include all known pyrrolidones that may serve as a softening agent and that may be used in implants to be fitted in the body. Examples of these pyrrolidones include alkyl or cycloalkyl substituted pyrrolidones, such as N-methyl-2-pyrrolidone (NMP), 1-ethyl-2-pyrrolidone (NEP), 2-pyrrolidone (PB) and 1-cyclohexyl-2-pyrrolidone (CP).

The plate 1 comprises an upper surface 2 and a lower surface 3. The lower surface 3 is intended for fitting against the bone and the upper surface 2 away therefrom, respectively. The upper and lower surfaces 2 and 3 may be planar or curved both in the longitudinal direction L of the plate and in the direction perpendicular thereto. The shape of the surfaces 2 and 3, as well as the other shapes of the plate may naturally differ from those shown in the figures.

The plate 1 comprises two parts: a first part 9 and a second part 10, between which there is an intermediate part 11. Hereinafter the first part 9 will be called a distal part 9 and the second part 10 will be called a proximal part 10, respectively. The distal part 9 is intended for attaching to one or more distal fragments off the radius bone due to a fracture or osteotomy or the like, whereas the proximal part 10 is intended for attaching to the shaft of the radius bone. The intermediate part 11 is thus located on the most typical fracture area or in the immediate vicinity thereof. The distal part 9 is arranged at an angle A to the proximal part 10. The angle A is now about 20 degrees, but it may vary within the range of 0° ... 45°, however. It should be noted that in the radius bone application the angle is advantageously between 0° ... 45°. In other plate applications the angle A may vary within the range of -90° ... 90° degrees.

In the upper surface 2 of the plate there are provided preholes 4. In this case the prehole 4 is a rotationally symmetrical, cylindrical through hole extending from the upper surface 2 to the lower surface 3 and whose imaginary longitudinal central axis is arranged perpendicularly to the lower surface 3. There are five preholes 4 in the distal part 9 and four preholes 4 in the proximal part 10. The number of the preholes 4 in the plate may vary. In the mouth of the prehole 4 there is provided a recess 6, which is not necessary, however.

The plate 1 comprises two preholes 4 aligned in the transverse direction of the plate in the end of the proximal part 10 which is closest to the distal part 9. By means of said holes it is possible to provide the plate with the firmest possible attachment close to the fracture line. It is obvious that there may be more than two holes side by side and that in the end of the distal part 9 which is closest to the proximal part 10 it is also possible to arrange two or more holes side by side.

The distal part 9 also comprises one free hole 5 which is a rotationally symmetrical, cylindrical through hole extending from the upper surface 2 to the lower surface 3, and whose imaginary longitudinal central axis is arranged perpendicularly to the lower surface 3. The diameter of the free hole 5 is larger than that of the prehole 4. The free hole 5 may also be arranged in the proximal part 10 or there may be free holes both in the distal and the proximal parts 9, 10.

The proximal part 10 comprises an elongated free hole 7 having a diameter that is larger in one direction than in the other direction and whose smallest diameter is larger than the diameter of the preholes. The longitudinal direction of the elongated hole is arranged to be parallel with the longitudinal direction L of the plate.

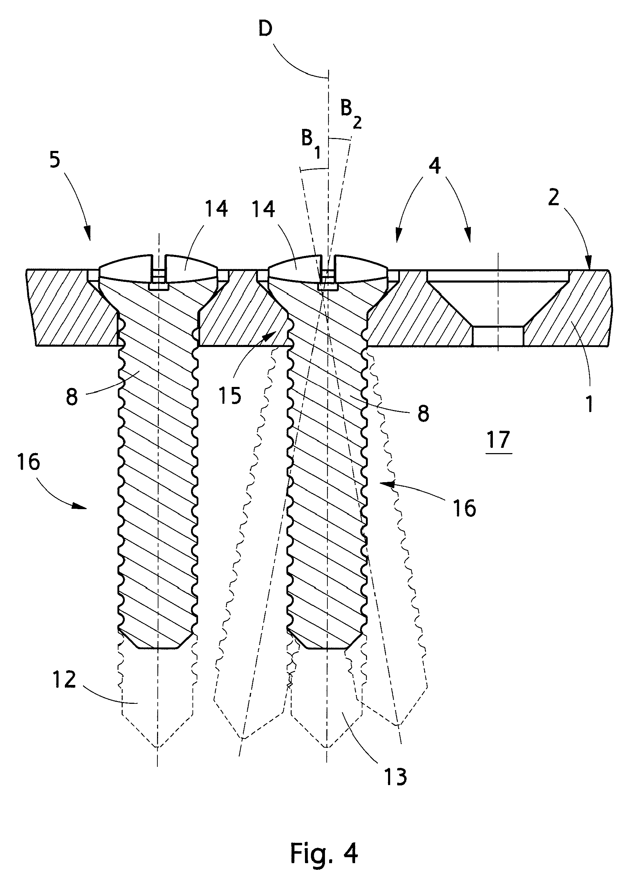

Figure 4 shows schematically a detail of the system in accordance with the invention in partial cross section. The plate 1 is attached to the bone fragments to be fixed such that its distal part 9 is attached to the distal part of the bone with an attachment screw 8 to be fitted through a free hole 5. It should be noted that hereinafter the attachment screw will be referred to as the screw.

The screw 8 is fitted in the free hole 5 by first arranging the distal part 9 in a suitable manner on the surface of the distal part of the bone. The plate 1 may be manufactured of material that allows bending or the like shaping to fit the surface of the bone. This material may be shaped at an elevated temperature but it is substantially rigid at body temperature. Said materials may be biostable or degradable in the body. The plate 1 made of this material may be heated in water bath, for instance, to a desired elevated temperature, whereafter the plate 1 will be placed on the surface of the bone and moulded to fit its contours. The elevated temperature may be for instance within the temperature range of 50°C ... 100°C. Advantageously the temperature is within 50°C ... 60°C for plates made of polylactide-co-trimethylene carbonate, 60°C ... 70°C for plates made of poly(L-lactide-co-D-lactide) and 70°C ... 90°C for plates made of poly(L-lactide). When necessary the plate 1 is shaped by bending to fit the contours of the bone surface.

Thereafter a pre-attachment hole 12 comprising a threaded bone part 16 is prepared in the bone through a free hole 5. The threaded bone part denotes a screw thread provided in the bone tissue and made, for instance, by drilling first an unthreaded hole in the bone through the free hole 5 and providing it thereafter with said thread, or by using a combination of a drill and a threading tap, i.e. the so-called self-tapping drill. It comprises a drilling bit and a threading thread part. In other words, the hole and its thread are made simultaneously by the same rotary motion of the self-tapping drill. A self-tapping drill of this kind is disclosed in US patent application No. 2004/0092950. It is also possible to use a so-called packing drill, which does not remove bone, but packs it tightly around the drill hole. Naturally it is also possible to use a combination of the packing, or at least partly packing, drill and the threading tap.

The largest diameter of the threaded bone part 16 is smaller than the smallest diameter of the free hole 5. After making the pre-attachment hole 12 the screw 8, whose threaded portion matches the thread of the pre-attachment hole, is driven through the free hole 5 into the pre-attachment hole 12. The outer diameter of the threaded portion of the screw 8 is smaller than the smallest diameter of the free hole such that the screw 8 is able to turn freely in the free hole 5. The screw-head 14 presses the plate 1 tightly against the bone.

The plate 1 may be rotated around the screw 8 fitted in the free hole 5 if it is necessary to adjust the position of the plate 1 in this way.

Next, the proximal part 10 is fitted at least approximately to its proper place on the surface of the radius bone and a second attachment hole is prepared in the radius bone through the elongated free hole 7. The preparation of the second pre-attachment hole may be carried out in the same manner as described above.

The position of the proximal part 10 may be adjusted in relation to the radius bone at least in the longitudinal direction of the elongated free hole 7 and also to some extent by rotating the plate in relation to the screw in the second pre-attachment hole.

Subsequently the distal bone part is placed exactly in correct position in relation to the radius bone, because the free hole 5 and the elongated free hole 7 enable fine adjustment of the position. When the bone fragments are in their proper places and when the screws 8 in the free hole 5 and in the elongated free hole 7 have been tightened, if so needed, the actual attachment holes 13 are prepared *in situ* in some of the preholes, or in some case in all preholes 4. In other words, the actual attachment holes 13 are prepared when the plate 1 is in its final position on the surface of the bone fragments to be fixed together. In this connection it should be noted that the elongated free hole 7 is not necessary for the implementation of the invention.

It is also possible that the attachment of the plate 1 to one bone fragment is accomplished, in other words, all screws to be secured to said bone fragment are driven into place prior to attaching the plate 1 to other bone fragments. The specialists participating in the attachment operation of the plate 1 decide case-specifically, and in view of the patient's needs, how the plate 1 is attached to its place and in which order the screws securing the plate 1 are driven.

The screw fitted in the free hole 5 is left in place in the pre-attachment hole 12.

The actual attachment holes 13 are prepared, for instance, as described in the following. First, a hole of convenient size is drilled via a prehole 4 through the plate 1 all the way to a required depth inside the bone. The drill may remove material also from the plate 1. The manufacturing material of the plate 1 enables the preparation of the attachment hole and the treaded portions thereof advantageously with instruments used for preparing bone holes, for instance. Thereafter said hole will be provided with a thread by using a threading tap or a like threading instrument. As a result there is provided an attachment hole 13 consisting of a threaded plate portion 15 in the plate 1 and a threaded bone portion 16 in the bone, which are mutually parallel and concentric. Thus, in the attachment hole 13 there is a continuous thread extending from the plate 1 to the bone. In the preparation of the actual attachment holes 13 it is possible to use the above-mentioned combination of a drill and a threading tap, or a packing drill, or a combination of a packing, or at least partly packing, drill and a threading tap.

In this connection it should be noted that the plate 1 may be attached also such that it is first attached to the bone by means of an actual attachment hole to be provided in a first prehole 4. Thereafter it is possible to proceed either by attaching the plate 1 to the bone with a screw to be fitted in a free hole 5, 7 or with a screw to be secured in a second prehole 4.

The prehole 4 facilitates the attachment of the plate 1 to the bone 17, because it forms on the upper surface side 2 of the plate a distinct starting point at which the preparation of the attachment hole 13 may be started.

The attachment hole 13 may be prepared such that its longitudinal axis is parallel with the longitudinal central axis D of the prehole 4. The attachment hole prepared in the attachment hole 13 appearing in Figure 4 has been oriented in this manner.

Alternatively, the attachment hole 13 is prepared such that the longitudinal axis of the attachment hole is not parallel to the longitudinal central axis D of the prehole 4, but it is at an angle B thereto. The value of angle B may be, for instance, within the range of >0° ... 30° or even more. Figure 4 shows two examples, B₁ and B₂ both now of 10°, how the longitudinal axis of the attachment hole may be oriented in relation to the central axis D of the prehole. The magnitude of angle B and thus the angle of the attachment hole 13 and the screw 8 to be fitted therein in relation to the plate 1 may be selected case-specifically in the most appropriate manner. Angle B may be divided into two orthogonal components which are projections thereof in the longitudinal direction L of the plate and perpendicular thereto in the transverse direction. In other words, the central axis of the attachment hole 13 may form an angle that differs from the orientation of the prehole 4 both in the longitudinal direction L and in the transverse direction of the attachment plate.

After accomplishing the attachment hole 13 a screw 8 may be driven therein. The screw 8 engages to the threaded portions 15, 16 of the attachment hole 13 such that a rigid attachment is provided between the screw 8 and the plate 1. The rigid attachment denotes that there exists no substantial macromotion, or angular motion, in the screw 8 in relation to the plate 1. The rigid attachment advantageously allows appropriate micromotion between the screw and the plate. The micromotion enhances fixation of bone fragments. Whereas a conventionally used metal plate does not allow micromotion, or allows it considerably less.

It is advantageous to prepare the attachment holes 13 such that the screws 8 do not attach to the bone in the same direction, because the screws 8 having different orientations lock the plate more firmly to the bone.

When the correct position in relation to the plate 1 is selected for the attachment hole 16, and the screw 8 to be fitted therein, it is possible to vary not only the angle of the attachment hole 16 to the plate 1, i.e. angle B, but also the location in which the mouth of the attachment hole 13 is prepared in the prehole 4. The mouth of the attachment hole 16 need not necessarily be arranged in the centre of the prehole 4, i.e. symmetrically to the prehole 4, but it may be offset in the longitudinal L or transverse direction of the plate 1, i.e. asymmetrically to the prehole 4. In Figure 4 the mouth of the attachment hole to be prepared at an angle B₁ is in the centre of the prehole 4, whereas the mouth of the attachment hole to be prepared at an angle B₂ is arranged asymmetrically to the prehole.

The surgeon in charge of attaching the plate 1 has versatile options to arrange each screw 8 to be fitted in the plate 1 individually in the exactly correct position in relation to the plate 1 and the bone fragments to be fixed.

Unlike the screws shown in Figure 4 the thread in the threaded portion of the screw 8 may be variable such that the crest of the screw 8 is higher in a portion closer to the tip of the screw 8 than in a portion further away from the tip. This screw has an advantage that it can be attached optimally both to the cortical bone and to the spongy bone therebeneath.

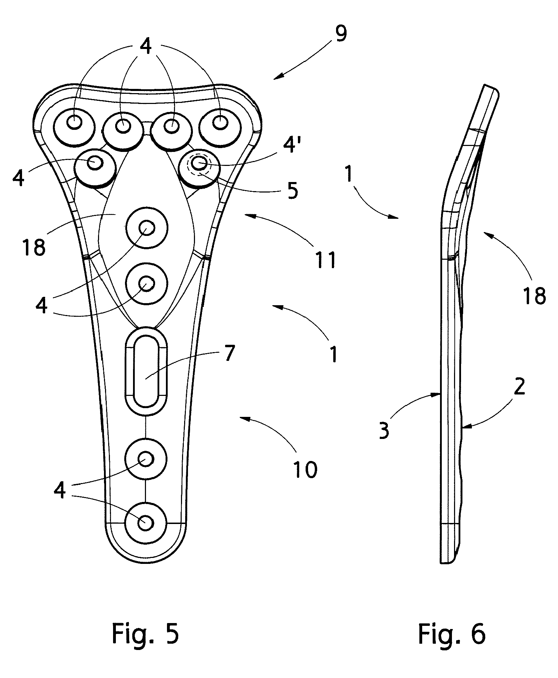

Figure 5 shows schematically a second plate in accordance with the invention seen from the direction of the upper surface. The plate 1 comprises ten preholes 4 in all, which are grouped in a suitable manner on the upper surface of the plate 1 and which extend through the plate 1 to the lower surface thereof. It is obvious that the number of the preholes may vary in the plates 1 in accordance with the invention. In addition, the plate 1 comprises one elongated free hole 7. The plate 1 may comprise a plurality of elongated free holes 7. The plate 1 further comprises a distal part 9, a proximal part 10 and an intermediate part 11 therebetween.

In the plate 1 of Figures 5 and 6 there is not yet any other free holes apart from the elongated free hole 7. If need be, a second, and optionally a third, fourth, etc. free hole 5 may be provided in the plate 1, for instance, by way of drilling in that stage of the plate mounting operation when the oprating surgeons have determined the most advantageous location and position for it in the plate 1. Figure 5 shows in broken lines one optional location for the free hole 5 in the plate 1. The preparation of said free hole 5 would utilize a prehole 4'. The smallest diameter of the free hole 5 is larger than the diameter of the prehole 4'. The free hole 5 may be prepared at an angle that differs from that of the prehole 4 in relation to the plate 1. Moreover, it should be noted that the free hole 5 may be provided in any one of the preholes 4 or even at a point in the plate 1 where there is no prehole 4, 4'. It should be noted that it is not necessary to prepare a second prehole 5, but the plate 1 may be attached by using the elongated free hole 7 as the only free hole.

The plate 1 shown in Figure 5 comprises a reinforcement 18 which is thicker than the rest of the plate and which extends from the distal part 9 through the intermediate part 11 to the proximal part 10. The reinforcement 18 is an oval, convex protrusion in shape, which is arranged on the upper surface of the plate 1. The reinforcement 18 reinforces the structure of the plate 1. In its preferable embodiment the reinforcement 18 does not extend to the edges of the plate 1, but the edge areas of the plate 1 are relatively thin throughout, whereby soft tissue can be arranged without problems over the plate 1.

The prehole 4 may also be a bottom hole provided in the upper surface 2 of the plate and extending to a distance from the lower surface 3 of the plate. Apart from being a circular cylinder, the prehole 4 may also be conical, and not necessarily perpendicular to the upper or lower surfaces 2, 3 of the plate. For instance, the prehole may be a tri- or polyhedral pyramid, a recess having the shape of a part of a spherical surface or the like. The one and the same plate 1 may comprise mutually different preholes 4.

The two preholes 4 arranged from the elongated free hole 7 in the direction of the distal part 9 are arranged successively in the longitudinal direction of the plate 1. This arrangement of the preholes 4 reinforces the plate 1 and enables a better positioning of the fracture line or osteotomy in relation to said preholes 4. The plate 1 may be arranged such that the fracture line or the osteotomy will be located in the area between said preholes 4 or between said preholes 4 and the preholes 4 of the distal end 9.

The screws to be fitted in the free hole 5 and in the actual attachment holes 13 are advantageously identical. Thus it is possible to reduce the number of components relating to the use of the plate 1.

The preholes 4 and the free holes 5 need not necessarily be perpendicular to the upper and lower surfaces 2, 3 of the plate 1.

In some cases the features presented in this document may be used as such, irrespective of other features. On the other hand, the features presented in this document may be combined, when necessary, to provide various combinations.

The drawings and the relating specification is only intended to illustrate the inventive idea. The details of the invention may vary within the scope of the claims.

## Claims

1. A plate for fixing bone fragments to one another, the plate (1) comprising
a first part (9) intended for attaching to at least one or more first bone fragments and a second part (10) intended for attaching to one or more second bone fragments,
an upper surface (2) and a lower surface (3), **characterized in that**
the upper surface (2) comprises a plurality of holes, of which some are preholes (4), in at least one of which preholes an actual, threaded attachment hole (13) is arranged to be prepared *in situ,* and
at least one free hole (5, 7), which is a through hole extending from the upper surface (2) to the lower surface (3), the smallest diameter of the free hole (5,7) being larger than the diameter of the preholes (4).

2. The plate of claim 1, **characterized in that** at least some of the preholes (4) are through holes.

3. The plate of claim 1 or 2, **characterized in that** at least some of the preholes (4) are bottom holes.

4. The plate of any one of the preceding claims, **characterized in that** the preholes (4) are round holes.

5. The plate of any one of the preceding claims, **characterized in that** the first part (9) is arranged at an angle (A) to the second part (10).

6. The plate of claim 5, **characterized in that** the angle (A) is -90° ... 90°.

7. The plate of any one of the preceding claims, **characterized in that** the largest width of the first part (9) is larger than the largest width of the second part (10).

8. The plate of any one of the preceding claims, **characterized by** comprising two preholes (4) aligned in the transverse direction of the plate at the end of the second part (10) that is closest to the first part (9).

9. The plate of any one of the preceding claims, **characterized in that** the free hole is an elongated free hole (7) whose smallest diameter is larger than the diameter of the preholes (4).

10. The plate of claim 9, **characterized in that** the longitudinal direction of the elongated free hole (7) is parallel to the longitudinal direction (L) of the plate.

11. The plate of any one of the preceding claims, **characterized by** being made of polymer material.

12. The plate of any one of the preceding claims, **characterized by** being made of biodegradable material.

13. The plate of any one of the preceding claims, **characterized by** being formable to match the contours of the bone fragments.

14. Use of the plate of claim 1 for fixing a distal radial fracture or osteotomy.

15. A system for fixing bone fragments to one another, **characterized by** comprising
a plate (1), which comprises a first part (9) intended for attaching to at least one first bone fragment, and a second part (10) intended for attaching to at least one second bone fragment,
an upper surface (2) and a lower surface (3), the upper surface comprising a plurality of holes, of which holes some are preholes (4), and
at least one free hole (5,7), which is a through hole extending from the upper surface (2) to the lower surface (3), the smallest diameter of the free hole (5,7) being larger than the diameter of the preholes (4), and the system further comprising
an attachment screw (8) comprising a threaded portion, whose outer diameter is at most equal in size with the smallest diameter of the free hole (5,7) but larger than the diameter of the prehole (4).

16. The system of claim 15, **characterized in that** the actual attachment hole (13), which extends through the plate (1) from the upper surface (2) to the lower surface (3), is arranged for being prepared in the plate via the prehole (4) such that the actual attachment hole (13) is arranged for being prepared *in situ* in the same operational step as the attachment hole portion to be provided in the bone.

17. The system of claim 16, **characterized in that** the actual attachment hole (13) is arranged to be provided with a thread such that the threaded portion (15) to be provided in the plate (1) is prepared in the same operational step as the threaded portion (16) to be provided in the bone, and that the attachment screw (8) is arranged to be fitted in the threaded portion (15) in the plate (1) and the threaded portion (16) in the bone such that the attachment screw (8) locks to the plate (1).

18. A method for fixing bone fragments to one another **characterized by** employing a plate (1), which comprises a first part (9) to be attached to at least one first bone fragment, and a second part (10) to be attached to at least one second bone fragment,
an upper surface (2) and a lower surface (3), the upper surface comprising a plurality of holes, of which holes some are preholes (4), and
at least one free hole (5,7), which is a through hole extending from the upper surface (2) to the lower surface (3), the smallest diameter of the free hole (5,7) being larger than the diameter of the preholes (4), and
attachment screws (8) comprising a threaded portion, whose outer diameter is at most equal in size with the smallest diameter of the free hole (5,7) and
by arranging the plate (1) on the bone,
providing the bone, through the free hole (5,7), with a preattachment hole (12) comprising a threaded portion (16) provided in the bone,
fitting the attachment screw (8) through the free hole (5,7) in the preattachment hole (12),
providing one or more preholes (4) with an actual attachment hole (13), which comprises the threaded plate portion (15) and the threaded bone portion (16) which is parallel and coaxial therewith and which extends inside the bone, and
driving the attachment screw (8) in the attachment hole (13).

19. The method of claim 18, **characterized by** providing the actual attachment hole (13) at an angle to the plate (1), which angle is different from the angle of the prehole (4) to the plate.

20. The method of claim 18, **characterized by** providing the actual attachment hole (13) at an angle to the plate (1), which angle is equal to the angle of the prehole (4) to the plate.

21. The method of claim 19, **characterized in that** the direction of the actual attachment hole (13) differs >0°...30° from the angle of the prehole (4) to the plate.

22. The method of any one of claims 18 to 21, **characterized by** selecting the location of the free hole (5) in the plate only in the course of the operation and preparing the free hole (5) in the location concerned.

23. The method of claim 22, **characterized by** preparing a free hole (5) in a prehole (4').

24. The method of any one of claims 18 to 23, **characterized by** using the plate for fixing a distal radial fracture or osteotomy.

25. The method of claim 24, **characterized in that** the first part (9) of the plate is the distal part and the second part (10) is the proximal part.

26. The method of claim 25, **characterized in that** the free hole (5, 7) is located in the distal part.

27. The method of claim 25, **characterized in that** the free hole (5,7) is located in the proximal part.
